(19) **Europäisches Patentamt**
**European Patent Office**
**Office européen des brevets**

(11) **EP 2 803 976 B1**

(12) **FASCICULE DE BREVET EUROPEEN**

(45) Date de publication et mention
de la délivrance du brevet:
**28.10.2015 Bulletin 2015/44**

(51) Int Cl.:
**G01N 21/53** *(2006.01)*    **G01N 33/00** *(2006.01)*
**G01N 21/05** *(2006.01)*    **G01N 21/03** *(2006.01)*
**G01N 21/3504** *(2014.01)*    **G01M 15/10** *(2006.01)*

(21) Numéro de dépôt: **14168055.3**

(22) Date de dépôt: **13.05.2014**

(54) **Dispositif et procédé de mesure de l'opacité et des oxydes d'azote dans les gaz d'échappement**

Messvorrichtung und Messverfahren der Rauchdichte und der Stickoxide in Abgasen

Device and process for measuring the opacity and the nitrogen oxides in exhaust gases

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **14.05.2013 FR 1354325**

(43) Date de publication de la demande:
**19.11.2014 Bulletin 2014/47**

(73) Titulaire: **Capelec Sarl
34000 Montpellier (FR)**

(72) Inventeurs:
- **Coton, Thierry
  34000 MONTPELLIER (FR)**
- **Bacher, Christian
  34000 Montpellier (FR)**
- **Ducros, Nicolas
  34000 Montpellier (FR)**
- **Martines, Nils
  34000 Montpellier (FR)**
- **Petelet, Georges
  34000 Montpellier (FR)**

(74) Mandataire: **Ipside
6, Impasse Michel Labrousse
31100 Toulouse (FR)**

(56) Documents cités:
  **EP-A1- 2 179 691**      **EP-A2- 1 176 412**
  **WO-A1-2013/057460**    **WO-A2-2011/143304**
  **GB-A- 1 200 038**      **US-A1- 2012 170 043**
  **US-A1- 2013 047 702**

- **"LE CONTROLE ANTIPOLLUTION DES
  MOTEURS DIESEL, PRINCIPE SIMPLE,
  APPLICATIONS COMPLEXES", REVUE
  TECHNIQUE AUTOMOBILE, ETAI, BOULOGNE-
  BILLANCOURT, FR, vol. 51, no. 580, 1 janvier 1996
  (1996-01-01), pages XIV-XXV, XP000555650,
  ISSN: 1621-3815**

**Description**

La présente invention appartient au domaine du contrôle des émissions polluantes par un véhicule automobile à moteur thermique, et plus particulièrement au domaine des dispositifs de mesure des matières polluantes présentes dans les gaz émis par la ligne d'échappement.

Elle a pour objet un dispositif de mesure qui permet de réaliser des mesures combinées de la teneur en oxydes d'azote (ou NOx) dans les gaz d'échappement, et de leur opacité laquelle est représentative de la teneur en particules.

Un moteur thermique émet durant son fonctionnement divers polluants, dont des particules solides de différentes tailles, majoritairement des particules de suie responsables de la formation des fumées noires (aussi appelées Black Carbon). Le flux gazeux émis comprend également différentes molécules à l'état gazeux dont la nocivité nécessite le contrôle strict. Ce sont en particulier les oxydes d'azote, qu'il s'agisse de monoxyde d'azote NO ou de dioxyde d'azote $NO_2$.

C'est pourquoi la législation qui faisait obligation de longue date de réduire les émissions de fumée noire par les moteurs Diesel, va imposer désormais également le contrôle du niveau des NOx en sortie d'échappement. Pour respecter les nouvelles normes Euro 5 et Euro 6, tous les véhicules neufs sont d'ores et déjà équipés d'un filtre à particules.

Le contrôle de la teneur en particules émises dans les gaz d'échappement est communément effectué par opacimétrie, qui consiste à mesurer l'opacité (inverse de la transparence) des gaz issus de la combustion des moteurs Diesel. Les centres de contrôle technique et les garages utilisent des opacimètres répondant aux normes actuellement en vigueur.

Les opacimètres sont communément constitués d'une chambre de mesure dans laquelle les gaz d'échappement circulent. La chambre est munie d'une source lumineuse à une extrémité et d'un récepteur à l'autre extrémité. L'intensité du faisceau lumineux traversant la chambre lorsqu'elle est remplie du gaz dont l'opacité est mesurée, décroît en proportion de l'opacité des gaz, c'est-à-dire de leur coefficient d'absorption conformément à la loi de Beer-Lambert, qui s'énonce par la relation :

$$I_R / I_S = e^{-K.L}$$

d'où on déduit l'opacité : $N = 100 (e^{-K.L})$

avec N : opacité (en %)

K : coefficient d'absorption (en $m^{-1}$)

L : longueur de gaz d'échappement traversé par le faisceau lumineux (en m)

$I_R$ : intensité lumineuse de l'émetteur

Is : intensité lumineuse reçue par le récepteur

De cette mesure d'opacité, il est aisé de déterminer la teneur en particules de fumées noires dans le volume gazeux, la proportion de lumière atteignant les moyens de réception étant inversement proportionnelle au taux de particules en suspension dans le gaz traversé. Un tel dispositif de mesure est connu par exemple des demandes de brevet FR-2 703 460 A1 et WO-2011/143304 A2. En France, on se référera à la norme R-10.025.3 qui définit la procédure de contrôle de l'opacité des gaz d'échappement.

Au cours des vingt dernières années, les législations ont durci les limites des émissions des véhicules neufs : division par 15 des émissions NOx et par 35 des émissions de particules entre la norme Euro 1 (1993) et Euro 6 (2014). Les nouvelles normes exigent cependant un saut quantitatif important dans la précision des mesures réalisées, ce qui est difficilement réalisable avec les équipements actuels qui ne sont pas en adéquation du point de vue de la précision, de la sensibilité et du niveau minimum devant être détecté en relation avec une gamme de mesure plus faible compatible avec les nouvelles législations d'émission. Au demeurant, les émissions de NOx n'étaient contrôlées jusqu'à présent que sur les moteurs à essence.

La mesure d'oxydes d'azote dans les gaz d'échappement (des moteurs à essence) se fait quant à elle communément à l'aide d'un capteur électrochimique. Son principe repose sur la mesure de la quantité d'oxygène produite par la réduction des NOx à l'aide d'un catalyseur adéquat. Les NOx doivent d'abord être amenés par pompage et séparées du reste des gaz par filtration. Les capteurs électrochimiques standards doivent être remplacés régulièrement et une calibration avec un gaz étalon doit être faite chaque fois, ce qui nécessite un matériel spécifique (bouteilles de gaz étalon, manomètre, détendeurs, ou autre). Cette mesure n'est pas pratiquée pour les moteurs Diesel.

Une autre méthode peut être utilisée, basée sur une détection de l'absorption des NOx dans l'infrarouge. Elle nécessite d'amener le flux gazeux jusqu'à un analyseur comprenant une cellule de détection couplée à une cellule de référence contenant le ou les gaz purs. Une calibration régulière avec un gaz étalon est nécessaire. Cette technologie est peu employée du fait des interférences liées à l'humidité des gaz.

Outre les inconvénients inhérents à chacune des méthodes de mesure exposées ci-dessus, un nouveau défi

est apparu du fait que les deux types de polluants doivent être réduits de manière conjointe. En effet, si la législation précédemment en vigueur prévoyait une simple mesure de l'opacité pour déterminer le bon fonctionnement du véhicule et son respect de l'environnement, elle impose désormais également le contrôle des émissions d'oxydes d'azote. Dans le même temps, les normes d'émission qui fixent les limites maximales de rejets polluants seront beaucoup plus strictes ce qui demande un appareillage de précision accrue. Par exemple, entre 2000 et 2014, pour l'homologation des véhicules par les constructeurs, le taux admis de NOx émis est passé de 0,5 g/km à 0,08 g/km pour les moteurs à essence, et de 0,15 g/km à 0,06 g/km pour les moteurs Diesels. De même, le taux maximum de particules est désormais de 0,005 g/km, soit dix fois moins qu'en 2000 pour les Diesels, alors qu'il n'était pas réglementé pour les moteurs à essence.

Il devient donc nécessaire de mener une lutte simultanée et conjointe contre ces deux types de polluants.

Or, les exigences de dépollution en ce qui concerne respectivement les particules et les oxydes d'azote sont contradictoires, dans la mesure où la majorité des moteurs Diesel est équipée d'un dispositif de réduction des émissions polluantes, appelé vanne RGE pour "recirculation des gaz d'échappement" (ou EGR pour "exhaust gas recirculation"), qui a pour rôle de diminuer les rejets d'oxydes d'azote, mais ce faisant augmente la production de particules. Ce système, qui satisfait à moindre coût aux normes européennes, consiste à rediriger une partie des gaz d'échappement des moteurs à explosion dans le collecteur d'admission. Il a pour effet de ralentir la combustion du mélange et d'absorber une partie des calories, ce qui diminue la température de combustion. De plus, il limite la présence d'oxygène dans le cylindre.

Ces deux effets entraînent certes une diminution des oxydes d'azote dans les gaz émis, mais la combustion étant moins poussée, les particules solides sont moins dégradées. Pour trouver un compromis entre la diminution des oxydes d'azote et l'augmentation de particules, les gaz réinjectés sont refroidis. Le fait d'abaisser la température des gaz réinjectés diminue la production des NOx pour le même taux de recirculation des gaz (même ouverture de la vanne RGE). Un équilibre peut être prédéfini, par pilotage de l'ouverture ou de la fermeture de la vanne RGE en fonction du régime du moteur (accélération libre ou faible charge) sans contrôle de l'émission effective des NOx, conformément aux normes dites Euro 5.

Il peut aussi être recherché en permanence en fonction des différents régimes de fonctionnement du moteur et des émissions de NOx relevées dans la ligne d'échappement, grâce à un système de diagnostic embarqué, plus connu sous le nom de OBD (pour On Board Diagnostic) aux Etats-Unis et d'EOBD en Europe. Le système est conçu pour recevoir des informations sur le fonctionnement du moteur thermique et sur les émissions de polluants, à partir desquelles il commande les moyens de régulation de l'ensemble des composants du moteur thermique affectant les émissions de polluants durant son fonctionnement. Dans ce cas, la quantité d'oxydes d'azote émise est relevée en continu par une sonde NOx au niveau de la ligne d'échappement et cette mesure physique est utilisée par le calculateur de bord pour commander l'ouverture ou la fermeture de la vanne RGE.

Il est impératif donc de considérer la teneur en particules simultanément à la teneur en oxydes d'azote et d'opérer une balance entre ces deux teneurs qui évoluent différemment lors du fonctionnement du moteur thermique. Dans ce contexte, il devient primordial de pouvoir mesurer, lors des contrôles techniques des véhicules, à la fois les teneurs en oxyde d'azote et en particules solides, en même temps et sur un même volume gazeux dégagé à un régime donné du moteur.

Les dispositifs de mesure d'opacité et les dispositifs de mesure des NOx utilisés actuellement par les constructeurs automobiles pour l'homologation des véhicules ne sont pas adaptés à la mesure sur tous les véhicules, notamment les véhicules à moteur Diesel, dans les garages et les centres de contrôle technique. Ils réalisent au mieux deux mesures séparées, sur des gaz émis successivement. Ils ne permettent pas d'effectuer les mesures de teneur en particules et en oxydes d'azote sur un même volume gazeux. De ce fait, les mesures ne sont pas corrélées, et les résultats obtenus ne peuvent pas donner pas une information fiable dans le cadre du contrôle technique.

La présente invention a donc pour objectif d'offrir une technologie qui permette d'effectuer simultanément une mesure d'opacité (représentative de la teneur en particules des fumées noires) et une mesure de teneur en oxydes d'azote sur une même fraction gazeuse prélevée à partir d'une ligne d'échappement d'un véhicule automobile, ceci avec une précision répondant aux normes de pollution les plus récentes.

Un autre objectif de l'invention est de répondre aux normes devenant de plus en plus sévères et exigeant une mesure de plus en plus précise sur des teneurs en polluants de plus en plus faibles. Le dispositif devra permettre de fournir une mesure d'opacité adaptée aux niveaux d'émission des moteurs thermiques, notamment des moteurs Diesel, avec une précision améliorée. Egalement, la mesure des NOx doit être réalisée dans l'environnement agressif des gaz d'échappement (encrassement, température), sans dégradation du dispositif ni perte de sensibilité.

Un objectif de l'invention est aussi de permettre de contrôler le fonctionnement correct du moteur thermique et de ses systèmes de dépollution des gaz d'échappement en fonction de ces deux teneurs simultanément déterminées. En particulier est recherchée la possibilité de déterminer si un système antipollution est en défaut, par association, comparaison ou recoupement des mesures d'opacité et de teneur en oxydes d'azote en sortie d'échappement, avec des informations prélevées à partir du système électronique de diagnostic embarqué (EOBD ou OBD).

De manière corollaire, cette mesure doit pouvoir être réalisée avec un temps de réponse très court, inférieur à une seconde, pour permettre une interaction efficace et rapide avec le système électronique de bord.

Pour répondre à l'ensemble de ces exigences, a été conçu un dispositif permettant d'effectuer simultanément sur une même fraction gazeuse provenant de la ligne d'échappement d'un véhicule automobile, une mesure d'opacité et une mesure de la teneur en dioxydes d'azote, et ce avec un temps de réaction extrêmement court.

Plus précisément, la présente invention a pour objet un dispositif de mesure simultanée de l'opacité et de la teneur en oxydes d'azote d'un gaz d'échappement d'un véhicule automobile à moteur thermique, le dispositif comprenant une enceinte à paroi tubulaire de longueur Le déterminée, fermée à ses extrémités, présentant un conduit d'entrée et au moins un conduit de sortie pour la circulation dudit gaz dans l'enceinte, le dispositif comprenant :

a) des moyens d'émission d'un faisceau lumineux traversant l'enceinte dans sa longueur, des moyens de réception dudit faisceau lumineux ayant traversé l'enceinte, et des moyens de transformation de celui-ci en un premier signal électrique proportionnel à l'intensité lumineuse reçue, pour donner une mesure de l'opacité du gaz présent dans l'enceinte,

b) des moyens de détection des oxydes d'azote logés au moins partiellement dans une cavité ménagée dans la paroi de l'enceinte, hors du trajet dudit faisceau lumineux, délivrant un second signal électrique proportionnel à la quantité d'oxydes d'azote détectée, pour donner une mesure de la teneur en oxydes d'azote du gaz présent dans l'enceinte, et

c) des moyens de transmission à une unité centrale et de traitement desdits premier et second signaux électriques.

Le terme "gaz d'échappement" désigne le flux essentiellement gazeux émis par un moteur à combustion. Il s'agit d'un mélange complexe contenant aussi bien des corps à l'état gazeux que des particules solides entraînées dans le courant de gaz. Par convention, on utilisera par la suite ce terme au singulier pour désigner un mélange donné émis à un moment donné et ayant une composition donnée.

Lors du contrôle d'un véhicule, la ligne d'échappement du moteur est dérivée vers le dispositif de mesure, de sorte qu'une fraction au moins du gaz d'échappement pénètre dans l'enceinte et circule dans celle-ci avant d'en être évacuée. La double mesure d'opacité et de teneur en NOx est réalisée sur une fraction du gaz traversant l'enceinte à un instant donné. L'enceinte est commodément de forme allongée et de section circulaire, avec un axe longitudinal principal (plus loin désigné comme l'axe de l'enceinte), mais peut adopter d'autres formes équivalentes pour les fonctions recherchées, par exemple adopter une section carrée. Elle peut être équipée d'autres organes agencés sur la structure tubulaire de base.

Elle comporte notamment un conduit d'entrée du gaz, qui est facilement relié à la ligne d'échappement pendant le fonctionnement du dispositif, et au moins un conduit de sortie pour l'évacuation du gaz. On comprend que pour obtenir une mesure fiable de l'opacité du milieu gazeux, la circulation du gaz doit être régulière et suivre un trajet assez long. On privilégie avantageusement une position aussi éloignée que possible entre l'entrée et la sortie du gaz.

L'opacité est mesurée par la perte d'intensité d'un faisceau lumineux ayant traversé le volume gazeux présent dans l'enceinte à cet instant. De manière connue, une source émettrice de lumière envoie un faisceau dans l'enceinte, lequel est reçu par un récepteur. La signal électrique généré est proportionnel à l'opacité, mais aussi à la longueur du trajet optique à travers le gaz. La longueur Le de l'enceinte est donc déterminée et fixe.

L'enceinte comporte également dans sa paroi une niche dont la paroi délimite une cavité ouverte vers le volume intérieur de l'enceinte. Cette cavité permet d'accueillir les moyens de détection des oxydes d'azote, ou du moins une partie de ceux-ci, de sorte qu'ils sont en contact avec le flux gazeux circulant dans l'enceinte sans toutefois couper le faisceau lumineux et gêner la mesure d'opacité.

Les signaux électriques générés sont transmis à une unité centrale apte à les transformer pour donner la valeur d'opacité et de teneur en NOx relevées. Le fait d'effectuer ces mesures sur un même volume gazeux permet d'associer une teneur en particules avec une teneur en oxydes d'azote présentes dans un gaz d'échappement de composition donnée à un instant donné, ce qui ne serait pas le cas si ces deux mesures étaient réalisées séparément. Ceci permet de contrôler la pollution d'un véhicule automobile en faisant la balance entre deux polluants rejetés, à savoir, dans le cas présent, les particules solides et les oxydes d'azote.

Selon une caractéristique avantageuse du dispositif de mesure objet de l'invention, les moyens d'émission et de réception du faisceau lumineux sont disposés à une première extrémité de l'enceinte, et des moyens de réflexion dudit faisceau sont disposés à une seconde extrémité de l'enceinte, de sorte que faisceau lumineux émis traverse deux fois l'enceinte, selon une trajectoire aller et une trajectoire retour, avant d'atteindre les moyens de réception.

Ceci présente l'avantage de doubler la longueur du trajet lumineux et donc de réaliser une mesure de précision sensiblement doublée pour une enceinte identique. Le dispositif peut ainsi conserver ses dimensions standards, ce qui n'augmente pas l'encombrement du dispositif et lui conserve sa maniabilité. Il est toutefois possible, et même intéressant, d'allonger la chambre puisque dans ce cas, chaque centimètre supplémentaire contribue doublement à accroître la précision de la mesure.

Selon une autre caractéristique avantageuse de l'invention, l'enceinte comprend, dans le prolongement dudit au moins un conduit de sortie, une prise d'air munie d'un ventilateur pour créer un flux gazeux traversant l'enceinte perpendiculairement à son axe entre ladite prise d'air et ledit conduit de sortie. On crée ainsi un écoulement laminaire qui entraîne le gaz d'échappement vers la sortie. Le ventilateur permet donc de faire passer un flux d'air propre qui fixe une longueur effective de gaz traversé par le faisceau lumineux pour la mesure d'opacité.

Conformément à un mode de réalisation préféré de l'invention, l'enceinte comprend deux ensembles, chacun formé d'une prise d'air et d'un conduit de sortie en prolongement, lesdits ensembles étant placés de part et d'autre dudit conduit d'entrée et définissant une chambre de circulation des gaz de longueur définie Lc. Il n'est donc pas nécessaire de recourir à une pompe pour entraîner le gaz à travers l'enceinte vers la sortie du dispositif. De ce fait, le système s'affranchit de l'utilisation d'une pompe pour l'amenée des gaz. La pression des gaz d'échappement suffit pour amener un échantillon de gaz représentatif jusqu'à la chambre de mesure.

Selon une caractéristique particulièrement intéressante du dispositif objet de la présente invention, la longueur *Lc* de la chambre de circulation des gaz (gaz d'échappement et air ambiant) est inférieure à la longueur *Le* de l'enceinte, celle-ci comportant à ses première et seconde extrémités respectivement une première et une seconde zones terminales libres de gaz d'échappement. Le gaz d'échappement est alors empêché de se répandre aux extrémités de l'enceinte au-delà du flux laminaire. De ce fait, les éléments optiques placés aux extrémités de l'enceinte ne subissent pas d'encrassement. Avantageusement, le conduit d'entrée du gaz part d'une sonde de prélèvement reliée à la ligne d'échappement et débouche dans la chambre de mesure à égale distance de la première et seconde extrémité de l'enceinte.

De préférence, dans le dispositif de mesure selon l'invention, les moyens de réflexion dudit faisceau lumineux comportent un miroir plan placé perpendiculairement à l'axe de l'enceinte dans ladite seconde zone terminale de l'enceinte. Ce miroir renvoie le faisceau lumineux selon la même trajectoire dans le sens direct et dans le sens retour. Il est placé à proximité de la paroi extrême de l'enceinte sans qu'une protection supplémentaire soit nécessaire.

Quand les moyens d'émission et de réception sont placés à la première extrémité l'enceinte, ceux-ci peuvent être confondus en un dispositif émetteur-récepteur intégré. Cependant un tel dispositif intégré est cher et délicat à mettre en oeuvre d'un point de vue technique. Selon un mode préféré de réalisation de l'invention, ils peuvent aussi être séparés, ce qui a l'avantage d'être moins onéreux, à condition de surmonter un certain nombre de difficultés de conception et d'agencement spatial. La présente invention offre une solution de construction simple et bon marché, avec utilisation d'un miroir plan comme élément réfléchissant à la seconde extrémité de l'enceinte. Dans ce contexte, est proposé une disposition de la première zone terminale de l'enceinte, laquelle est conformée en caisson comportant des parois orthogonales entre elles, dans laquelle on trouve les moyens d'émission et de réception sur deux parois différentes, ainsi qu'une lame séparatrice qui est placée dans un plan orienté à 45° par rapport auxdites deux parois. Les lames séparatrices sont des objets connus en optique, qui filtrent une partie du faisceau incident, et en réfléchissent une autre partie, dans un rapport constant. De manière originale, la lame remplit ici sa fonction séparatrice aussi bien dans le sens directe que dans le sens retour du faisceau.

Ainsi, est objet de la présente invention un dispositif de mesure tel que décrit ci-dessus dans lequel les moyens d'émission et de réception sont disposés dans un caisson ménagé à la première extrémité de l'enceinte, sur deux parois dudit caisson orientées selon des plans perpendiculaires, et une lame séparatrice est placée dans un plan orienté à 45° par rapport auxdites deux parois sur la trajectoire directe et sur la trajectoire retour du faisceau lumineux, de sorte que chacun desdits faisceaux direct et retour est séparé en un faisceau transmis (ft) et un faisceau dévié (fd) d'intensité relative fixe. De cette manière, le faisceau direct émis ($F_D$) est en partie transmis vers le miroir, et en partie dévié dans une première direction, puis le faisceau retour ($F_R$) réfléchi par le miroir, se divise à son tour en une partie transmise (ft) qui reste dans la direction initiale, celle de l'émetteur et n'a donc pas d'effet, et en une partie déviée (fd) dans une autre direction pointant vers la positon du récepteur.

On peut mettre à profit cette disposition pour réaliser un contrôle de l'intensité du faisceau émis. Pour cela, selon un mode particulier de réalisation de l'invention, le caisson comprend un récepteur de référence placé dans l'axe du faisceau direct dévié, et des moyens de transformation de ce dernier en un signal électrique proportionnel à l'intensité lumineuse reçue. Le rapport d'intensité entre le faisceau transmis et dévié étant constant, commodément 50%, l'intensité reçue par le récepteur de référence est proportionnelle à l'intensité du faisceau direct émis. Ceci permet de vérifier l'état de l'émetteur pour corriger les valeurs d'opacité, ou le remplacer quand c'est nécessaire.

Dans un mode de réalisation avantageux du dispositif selon l'invention, les moyens de détection des oxydes d'azote comprennent un capteur NOx dont l'élément sensible est à base de céramique d'un oxyde métallique, muni d'une paire d'électrodes mesurant une différence de potentiel ou un courant proportionnel à la concentration en NOx dans le gaz d'échappement. Ce capteur à oxydes d'azote est avantageusement une sonde dite à technologie céramique. La sonde comprend une première cellule dans laquelle les dioxydes d'azote du gaz sont transformés en monoxydes d'azote et l'oxygène libre présent dans le gaz est éliminé. Dans une seconde cellule, les monoxydes d'azote sont décomposés par une électrode réductrice en diazote et en oxygène. La quantité d'oxygène produite est alors mesurée et donne la concentration des oxydes d'azote initialement présents dans le gaz.

C'est un point innovant du dispositif ici décrit d'utiliser pour analyser les NOx, une sonde qui a été conçue et qui est utilisée jusqu'à présent dans les systèmes embarqués de dépollution équipant les véhicules. Elle est usuellement placée dans la ligne d'échappement pour fournir la donnée d'entrée à la boucle de régulation électronique des systèmes embarqués. Elle a été conçue pour être utilisée dans des environnements très sévères (encrassement, température) tels que les gaz d'échappement et est adaptée à un emploi dans un dispositif de métrologie selon l'invention, dans lequel elle est intégrée pour la première fois.

Contrairement aux méthodes concurrentes qui utilisent d'autres technologies de mesure, le système selon l'invention s'affranchit des opérations de filtration nécessaires pour dissocier les particules solides des NOx avant de procéder à la mesure des NOx. Elle est beaucoup moins sensible à l'encrassement.

Un autre avantage très appréciable découlant du choix de ce type de capteur est qu'il ne nécessite pas de calibration avec un gaz étalon, comme ça peut être le cas avec des technologies de mesure conventionnelles (capteurs infrarouge, électrochimiques ou autre). Seule une re-calibration avec de l'air ambiant peut être nécessaire de temps à autre, qui peut être aisément automatisée. Elle est alors réalisée de manière complètement transparente pour l'utilisateur puisqu'elle ne nécessite aucune intervention particulière d'un technicien et qu'il n'est pas nécessaire non plus de disposer d'outils ou d'un matériel spécifique (bouteille de gaz étalon, détendeurs, manomètre, ...). Quoi qu'il en soit, même s'il s'avérait nécessaire de le remplacer, la calibration après remplacement serait faite automatiquement. Le remplacement de ce capteur ne nécessite donc aucune autre intervention. Au demeurant, elle a une durée de vie améliorée d'un rapport 50 par rapport aux solutions électrochimiques utilisées sur les analyseurs de gaz à technologie infrarouge.

Enfin, on pourra mettre à profit le fait que cette sonde a un temps de réponse très court (inférieur à une seconde) pour réaliser des mesures très rapprochées dans le temps, quasiment en régime continu. Ceci permettra de continuer à utiliser la procédure actuelle de test en contrôle technique pour les véhicules diesels (c'est-à-dire mesure des pics de pollution lors d'accélérations libres), chose que ne permettent pas les technologies dont le temps de réponse est de plusieurs secondes, comme les méthodes basées sur les mesures infrarouge. On pourra également, si désiré, avoir une mesure pertinente selon d'autres procédures de test qui nécessiteraient une certaine réactivité de la mesure, comme la mise en charge brève du moteur du véhicule.

Selon une caractéristique avantageuse de l'invention, ledit capteur NOx et ledit conduit d'entrée du gaz d'échappement sont placés à la partie médiane de l'enceinte, la cavité logeant le capteur NOx étant ménagée dans la paroi supérieure de l'enceinte et l'élément sensible dudit capteur NOx affleurant du plan de la paroi de l'enceinte, tandis que le conduit d'entrée débouche dans l'enceinte selon une orientation sensiblement horizontale. Par convention, on désigne "partie supérieure" celle qui occupe la position la plus haute lorsque le dispositif est en fonctionnement. On évite ainsi le risque de contaminer et d'endommager le capteur avec des projection d'eau provenant du gaz et on réduit encore son encrassement.

De préférence selon l'invention, le dispositif de mesure comporte des éléments chauffants thermostatés disposés sur une au moins des parties de l'enceinte suivantes :

- la paroi de l'enceinte,
- la paroi de la cavité logeant au moins partiellement le capteur NOx,
- la première et la seconde zones terminales de l'enceinte.

Les éléments chauffants ont une température prédéterminée et/ou régulée, de manière à empêcher que de la vapeur d'eau ne se dépose sur les parois froides.

Selon une caractéristique du dispositif de mesure selon l'invention, la paroi de l'enceinte est revêtue d'un matériau absorbant la lumière dans la bande de fréquence du visible et de la source lumineuse, afin qu'aucune réflexion ou transmission de lumière indésirable n'atteigne le récepteur.

Selon une caractéristique particulièrement intéressante du dispositif de mesure objet de la présente invention, les moyens de traitement desdits premier et second signaux électriques comprennent des moyens de stockage et d'impression de la valeur de l'opacité et de la teneur en NOx dudit gaz d'échappement, mesurées à un même instant de fonctionnement dudit véhicule automobile. Les valeurs mesurées des teneurs en oxydes d'azote et d'opacité sont transmises à des moyens de calcul et d'enregistrement dont le dispositif est doté, pour leur mémorisation. Des moyens de traitement, essentiellement électroniques permettent le traitement instantané ou différé de ces valeurs.

Dans la mesure où le contrôle des NOx n'était pas inclus dans le contrôle technique des véhicules jusqu'à présent, une nouvelle procédure peut consiste à vérifier que les niveaux de particules dans les gaz d'échappement restent inférieurs aux normes, ou bien que les organes de régulation pilotés par le système de diagnostic répondent correctement aux variations de régime du moteur pour optimiser le rapport particules/NOx à tout moment de son fonctionnement et aux différents régimes. Il n'est alors pas impératif de faire des contrôles de niveaux maximum, car il faudrait pour cela mettre le véhicule sur des bancs de puissance extrêmement onéreux. Une procédure selon l'invention, repose sur la mesure d'une différence entre d'une part un régime de ralenti et de ralenti accéléré, et d'autre part un régime de ralenti accéléré et des accélérations, ce qui n'impose pas d'acquérir un matériel annexe de type bancs de puissance.

Comme expliqué précédemment, les mesures peuvent se faire en continu, non seulement en ce qui concerne l'opacité, mais aussi en ce qui concerne la teneur en NOx, ce qui représente un avantage inédit du dispositif selon l'invention. En effet, de ce fait notamment, il est désormais possible grâce à la présente invention, de combiner une mesure d'opacité adaptée aux niveaux d'émission des moteurs et une mesure de NOx en sortie d'échappement, et de les recouper avec des informations prélevées depuis l'électronique embraquée. On peut ainsi mettre en oeuvre un protocole de mesure défini pour les moteurs classés "Euro 5", basé sur la recherche d'un écart prouvant une réactivité de la vanne EGR à des conditions de fonctionnement différentes détectées et gérées par le calculateur de gestion moteur.

Pour les moteurs répondant au niveau "Euro 6", le principe est plus simple. En effet ces moteurs sont dotés d'une sonde NOx dont les informations sont exploitées par le calculateur de bord (EOBD). Des données sur les teneurs en NOx émises aux différents régime du moteur sont donc accessibles via une connexion EOBD auprès du calculateur. Lors d'un contrôle technique, le protocole de mesure consiste à identifier si ces valeurs vues à partir calculateur sont homogènes et cohérentes avec la réalité (c'est-à-dire avec la mesure réalisée en sortie d'échappement). On opère par une comparaison entre information interne du véhicule et mesure physique en sortie de pot d'échappement.

Ainsi, selon une autre caractéristique intéressante du dispositif de mesure objet de l'invention, les moyens de traitement desdits premier et second signaux électriques comprennent des moyens pour comparer les valeurs d'opacité et de teneur en NOx mesurées avec des valeurs calculées par un système électronique régulant les émissions de NOx dudit véhicule automobile. On peut ainsi vérifier l'adéquation entre les valeurs relevées par le dispositif de mesure selon l'invention, avec les informations et les commandes données par l'ordinateur de bord du véhicule, lorsqu'il en est équipé.

Le dispositif selon l'invention trouve son utilisation dans les centres de contrôle technique des véhicules automobiles, lors du contrôle des polluants émis, ainsi que dans les garages ou en station-service, en France et à l'étranger. Il est transportable léger et adaptable à son environnement. Il peut aussi servir au contrôle des systèmes de dépollution présents dans la ligne d'échappement des véhicules.

La présente invention concerne donc aussi un procédé permettant de déterminer de manière rapide, fiable et commode pour l'opérateur, deux paramètres dont les niveaux sont réglementés et soumis au contrôle technique des véhicules.

Est également objet de l'invention, un procédé de mesure simultanée de l'opacité et de la teneur en oxydes d'azote d'un gaz d'échappement émis par un véhicule automobile à moteur thermique, le procédé comprenant les étapes consistant à :

- faire circuler ledit gaz d'échappement dans l'enceinte d'un dispositif de mesure simultanée de l'opacité et de la teneur en oxydes d'azote, le dispositif comprenant une enceinte à paroi tubulaire de longueur Le déterminée, fermée à ses extrémités et présentant un conduit d'entrée et au moins un conduit de sortie pour la circulation dudit gaz dans l'enceinte,
- émettre un faisceau lumineux à travers l'enceinte dans sa longueur depuis une source lumineuse jusqu'à un récepteur, pour fournir un premier signal électrique proportionnel à l'intensité lumineuse reçue,
- réaliser la détection des oxydes d'azote par des moyens de détection logés au moins partiellement dans une cavité ménagée dans la paroi de l'enceinte, hors du trajet dudit faisceau lumineux, délivrant un second signal électrique proportionnel à la quantité d'oxydes d'azote détectée,

pour obtenir une mesure de l'opacité du gaz d'échappement présent dans l'enceinte et une mesure de la teneur en oxydes d'azote dans ledit gaz présent dans l'enceinte à un même instant du fonctionnement dudit véhicule automobile.

Dans un mode de mise en oeuvre avantageux du procédé de mesure selon l'invention, le faisceau lumineux émis traverse deux fois l'enceinte, selon une trajectoire directe et une trajectoire retour, avant d'atteindre les moyens de réception. De préférence, le faisceau est réfléchi par un miroir plan, de sorte que la trajectoire directe du faisceau (faisceau direct $F_D$) à travers l'enceinte est identique à la trajectoire retour (faisceau retour $F_R$). On incorpore avantageusement et de manière connue des lentilles de collimation placées judicieusement sur le trajet optique, pour focaliser le faisceau lumineux d'une part sur le miroir, d'autre part sur le récepteur, et éviter ainsi sa dispersion vers les parois de l'enceinte.

Selon une caractéristique particulièrement intéressante de la présente invention, le gaz d'échappement circule dans l'enceinte depuis ledit conduit d'entrée jusqu'audit au moins un conduit de sortie par entraînement par un flux d'air qui est introduit dans l'enceinte à proximité de chaque conduit de sortie par une prise d'air pulsé par un ventilateur, pour créer un écoulement laminaire des gaz traversant l'enceinte perpendiculairement à son axe entre ladite prise d'air et ledit conduit de sortie.

Dans un mode de réalisation préféré du procédé de mesure selon l'invention, le gaz d'échappement circule dans l'enceinte depuis ledit conduit d'entrée jusqu'à deux conduits de sortie placés de part et d'autre dudit conduit d'entrée, de l'air étant introduit dans l'enceinte à proximité des deux conduits de sortie par deux prises d'air et pulsé par deux ventilateurs, pour créer deux écoulements laminaires qui traversent l'enceinte perpendiculairement à son axe et définissent une chambre de circulation des gaz de longueur définie *Lc,* inférieure à la longueur *Le* de l'enceinte. Les gaz (gaz d'échappement et air ambiant laminaire) circulent donc uniquement dans cette chambre de circulation, qu'on peut qualifier de virtuelle, de longueur fixe et définie par la position des conduits d'entrée de l'air pulsé dans l'enceinte, sans encrasser les éléments d'optique placés à l'une ou l'autre extrémité de l'enceinte.

Selon un mode de réalisation particulièrement intéressant de l'invention, le faisceau lumineux est émis par une source lumineuse et est reçu par un récepteur qui sont placés dans une première zone terminale ménagée à une première extrémité de l'enceinte, et le faisceau lumineux est réfléchi par un miroir plan qui est placé dans une seconde zone terminale ménagée à une seconde extrémité de l'enceinte, lesdites première et seconde zones terminales étant libres de gaz d'échappement.

Dans un mode de réalisation particulier du procédé selon l'invention, le faisceau direct $F_D$ est divisé avant de traverser l'enceinte par une lame séparatrice, en un faisceau transmis ft orienté dans l'axe de l'enceinte, et en un faisceau dévié fd orienté vers un récepteur de référence fournissant un signal proportionnel à l'intensité dudit faisceau dévié, lesdits faisceaux transmis et dévié ayant une intensité relative déterminée fixe.

Dans un autre mode de réalisation particulier du procédé selon l'invention, lorsque le faisceau lumineux retour $F_R$ a traversé l'enceinte, il est divisé par la lame séparatrice en un faisceau transmis et en un faisceau dévié qui est orienté vers les moyens de réception.

Selon une caractéristique préférée de l'invention, les oxydes d'azote sont détectés par un capteur NOx affleurant du plan de la paroi de l'enceinte, à proximité du conduit d'entrée des gaz d'échappement, lequel débouche dans l'enceinte dans sa partie médiane. On agence le capteur et le débouché du conduit d'entrée de façon à éviter toute projection risquant d'endommager le capteur, par exemple celui-ci peut être placé au-dessus du conduit d'entré du gaz.

De manière préférée et avantageuse, selon l'invention, les oxydes d'azote sont détectés avec un temps de réponse inférieur à une seconde. Le choix du capteur NOx tel que défini ci-dessus est déterminant à ce sujet. Cette caractéristique est primordiale pour une mesure simultanée des NOx et de l'opacité, les mesures pouvant être réalisées instantanément, à chaque changement de régime du moteur.

Selon l'invention, on empêche toute condensation sur les parois du dispositif. C'est pourquoi, de préférence, on maintient à une température contrôlée une ou plusieurs des parties l'enceinte suivantes : la paroi de l'enceinte, la paroi de la cavité logeant le capteur NOx, les zones terminales de l'enceinte.

De préférence également, la lumière pénétrant dans l'enceinte ou réfléchie par la paroi est absorbée par un matériau recouvrant ladite paroi.

Conformément à l'invention, lesdits premier et second signaux électriques sont transmis à une unité centrale et sont traités pour calculer l'opacité et la teneur en NOx dudit gaz d'échappement, mesurées à un même instant de fonctionnement dudit véhicule automobile. Elles sont stockés et imprimés (et/ou affichées), afin que l'opérateur puisse constater la conformité du véhicule ou recommander une intervention.

De manière avantageuse, conformément au procédé de mesure selon l'invention, lesdits premier et second signaux électriques sont transmis à une unité centrale et sont traités par comparaison des valeurs d'opacité et de teneur en NOx mesurées, avec des valeurs calculées par un système électronique régulant les émissions de NOx dudit véhicule automobile.

La présente invention sera mieux comprise, et des détails en relevant apparaîtront, grâce à la description qui va être faite d'une de ses variantes de réalisation, en relation avec les figures annexées, dans lesquelles :

La fig.1 est une vue schématique en coupe d'un dispositif de mesure selon l'invention.
Les fig. 2a et 2b sont des vues schématiques en coupe d'un dispositif selon l'invention, montrant le parcours optique du faisceau direct (fig. 2a) et du faisceau retour (fig. 2b).
Les fig. 3 et 4 illustrent des protocoles de contrôle des émissions selon les types de moteur, à l'aide d'un dispositif selon l'invention

EXEMPLE 1 : Dispositif de mesure simultanée

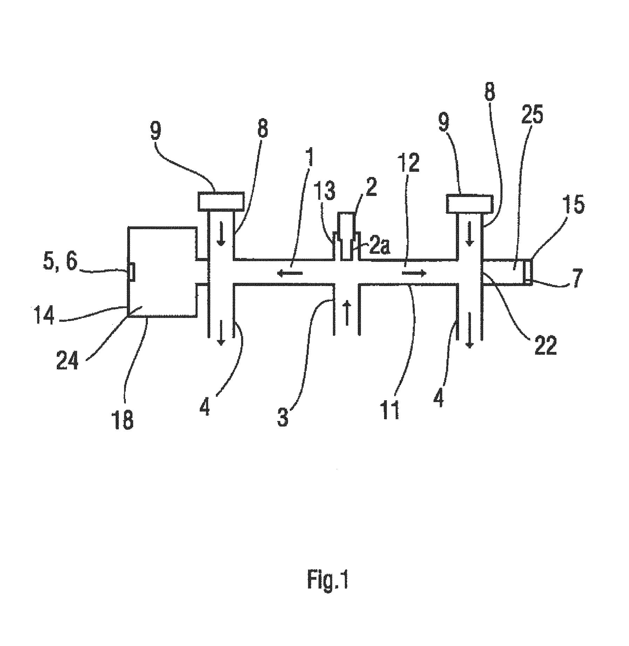

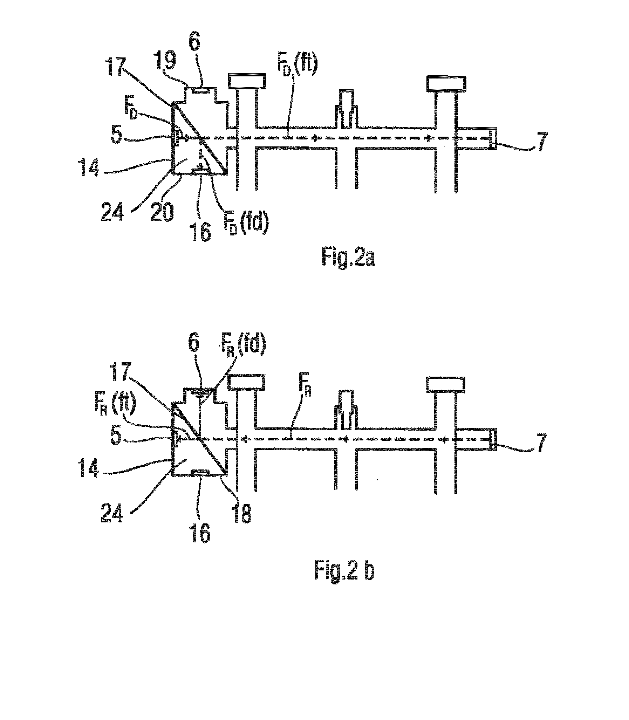

Sur la fig.1, on a représenté un dispositif de mesure simultanée de l'opacité et de la teneur en oxydes d'azote d'un gaz d'échappement d'un véhicule automobile à moteur thermique. Il comprend l'enceinte 1 à paroi tubulaire de longueur Le déterminée et fixe. La paroi tubulaire 11 peut avoir une section droite quelconque mais est plus commodément circulaire. L'enceinte 1 est fermée à ses extrémités 14, 15 par des cloisons d'extrémité. L'enceinte est munie à sa première extrémité 14 d'un moyen d'émission 5 d'un faisceau lumineux et d'un moyen de réception 6 du faisceau lumineux à la fin de son trajet optique, qui sont ici confondus en un dispositif émetteur-récepteur intégré (fig.1). Comme il sera décrit plus loin, on peut utiliser un émetteur et un récepteur distincts (fig. 2). Le récepteur est relié à un circuit électrique qui transmets le signal reçu, proportionnel à l'intensité lumineuse, à une unité apte à transformer le signal électrique en une valeur quantitative de l'opacité et de là une valeur de la teneur en particules solides.

Un miroir plan 7 est disposé à la seconde extrémité 15 de l'enceinte 1, de sorte que le faisceau lumineux émis traverse deux fois l'enceinte, selon la trajectoire directe $F_D$ puis une trajectoire retour $F_R$, avant d'atteindre le récepteur. Le faisceau lumineux a la même trajectoire à travers la chambre de mesure dans le sens direct et dans le sens retour.

L'enceinte 1 comporte une niche définissant une cavité 13 ménagée dans la paroi 11 supérieure de l'enceinte, à sa partie médiane. Dans la cavité est logé un capteur NOx 2, qui est conçu pour détecter les oxydes d'azote. Son élément sensible 2a est muni d'une paire d'électrodes mesurant une différence de potentiel proportionnelle à la concentration en NOx dans le gaz et délivrant un second signal électrique qui est transformé pour donner une mesure quantitative de la teneur en oxydes d'azote du gaz présent dans l'enceinte. On utilise une sonde disponible dans le commerce.

Le capteur 2 est placé dans le logement de manière que son élément sensible 2a affleure du plan de la paroi 11 de l'enceinte, sans couper le trajet dudit faisceau lumineux. Par ailleurs, le conduit d'entrée 3 débouche dans l'enceinte 1 au même niveau médian que le capteur NOx, mais selon une orientation sensiblement horizontale.

L'enceinte 1 présente un conduit d'entrée 3 qui est relié à la ligne d'échappement pendant le fonctionnement du dispositif, et deux conduits de sortie 4, par lesquels le gaz d'échappement est évacué. Une prise d'air 8 est ménagée dans le prolongement de chaque conduit de sortie 4. Chacune d'elle est munie d'un ventilateur 9 qui pulse de l'air ambiant vers l'enceinte et vers les conduits de sortie 4. On crée ainsi un flux gazeux traversant l'enceinte 1 perpendiculairement à son axe entre une prise d'air 8 et le conduit de sortie en regard, par écoulement laminaire et qui entraîne le gaz d'échappement vers la sortie. Cet écoulement est stable de sorte que les gaz s'écoulent dans une chambre de mesure virtuelle de longueur effective Lc constante et définie par la limite 22 des deux flux laminaires. La longueur de gaz traversée par le faisceau lumineux pour la mesure d'opacité est donc de deux fois la longueur Lc. L'enceinte comporte ainsi à ses première et seconde extrémités 14, 15, respectivement une première et une seconde zones terminales 24, 25, dans lesquelles le gaz d'échappement ne se répand pas. Par conséquent, le miroir plan 7, disposé à la seconde extrémité 15 de l'enceinte 1, reçoit le faisceau lumineux, sans être au contact des gaz. Il en va de même des éléments optiques (émetteur et récepteur notamment) placés dans la première zone à la première extrémité de l'enceinte. L'appareil peut être utilisé autant en extérieur qu'en intérieur et la gamme de température ambiante d'utilisation allant de 0°C à 50°C. La mesure peut se faire en continu.

Ce type de technologie permet de faire une mesure de NOx en ppm vol. dans la gamme [0 - 5000 ppm vol.]. La précision est :

$\pm$ 15 ppm de 0 à 1000 ppm,
$\pm$ 1,5% de 1001 ppm à 5000 ppm.

En ce qui concerne la mesure d'opacité en % ou en $m^{-1}$ dans la gamme [0 - 99.9%] ou [0 - 9.9%], on obtient une résolution de 0.1% ou 0,001 $m^{-1}$ (0,001 $m^{-1}$ si K < 0,50 $m^{-1}$ et 0,01 $m^{-1}$ si K > 0,50 $m^{-1}$). La précision est la suivante (pour une concentration par unité de volume déduite du nombre de particules mesuré) :

- Précision dynamique : 0,05 $m^{-1}$ (concentration équivalente : 10 $mg/m^3$)
- Précision du zéro : < 0,005 $m^{-1}$ (concentration équivalente : 1 $mg/m^3$)
- Précision statique : 0,5 % (filtre)
- Résolution : 0,001 $m^{-1}$ (0,001 %) si K > 0,50 $m^{-1}$

Ces résultats sont largement plus précis et fiables que ceux obtenus avec les méthodes conventionnelles, pour lesquelles on avait typiquement :

- Précision dynamique : 0,15 $m^{-1}$
- Précision du zéro : < 0,2 %
- Précision statique : < 1 %
- Résolution : 0,01 $m^{-1}$ si K > 0,50 $m^{-1}$

## EXEMPLE 2 : Dispositif avec émetteur et récepteur séparés

Dans cet exemple, les moyens d'émission 5 et de réception 6 sont disposés dans un caisson 18 ménagé à la première extrémité 14 de l'enceinte 1 sur deux parois dudit caisson orientées selon des plans perpendiculaires. L'émetteur 5 est placé dans l'axe principal de l'enceinte sur la paroi formant la cloison extrême 14, tandis que le récepteur est placé sur une première paroi latérale 19. Dans le caisson 18, se trouve une lame séparatrice 17 orientée à 45° par rapport aux deux parois, sur la trajectoire directe ($F_D$) et sur la trajectoire retour ($F_R$) du faisceau lumineux. Un récepteur de référence 16 est placé sur une seconde paroi latérale 20, opposée à la paroi 19 où se trouve de récepteur 6.

Le faisceau direct émis ($F_D$) est en partie transmis vers le miroir $F_D$(ft) à travers la chambre de mesure, et en partie dévié dans une première direction $F_D$(fd) où il va frapper récepteur de référence 16. Puis le faisceau retour ($F_R$) réfléchi par le miroir à travers la chambre de mesure, et se divise à son tour en une partie transmise $F_R$(ft) qui reste dans la direction initiale qui est celle de l'émetteur et n'a donc pas d'effet, et en une partie déviée $F_R$(fd) à angle droit vers le récepteur 6. Ce montage simple et peu onéreux ne nécessite pas de réglages complexes. Etant situé dans la zone terminale, il est en outre à l'abri des fumées. *In fine,* il est très précis, fiable et durable.

**EXEMPLE 3 : Compensation de la contribution des NOx à l'opacité**

La mesure d'opacité se correspond à la proportion de particules de suie dans le gaz. Ces particules de suie sont majoritairement contributives à l'opacité des fumées. Cependant, les émissions des moteurs diesel sont complexes, et différents gaz sont émis dont certains présentent une opacité significative. En particulier $NO_2$ étant un gaz brun-rouge, il pourrait fausser de très faibles valeurs d'opacité. Or, les nouveaux moteurs répondant aux évolutions de réglementation Euro 5 et Euro 6 présentent des valeurs d'opacité extrêmement faibles. Si la teneur en particules exprimée en $mg/m^3$, est d'un niveau équivalent à la contribution due au gaz $NO_2$ , la valeur d'opacité due aux particules doit être compensée.
Le dioxyde d'azote, $NO_2$, est en équilibre avec le tétraoxyde de diazote $N_2O_4$ incolore, selon l'équation :

$$2\ NO_2\ (g) <===> 1\ N_2O_4\ (g)$$

Cette compensation peut être initialisée grâce à un point de calibration obtenu par l'intermédiaire d'un gaz (bouteille étalon) à haute teneur en NO2. Cette calibration en un point permet ensuite d'apporter une compensation en opacité à partir de la valeur $NO_2$ mesurée.

**EXEMPLE 4 : Contrôle des moteurs Euro 5**

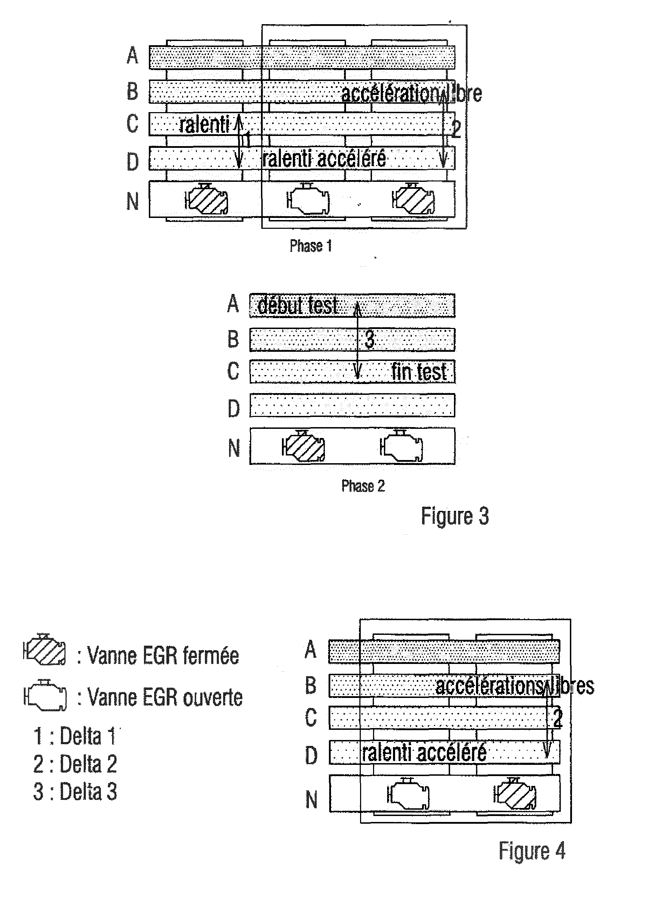

Dans le cas de moteurs Euro 5, il est tenu compte du comportement et du pilotage de la vanne EGR. Le processus de mesure pour les moteurs Euro 5 est basé sur la recherche d'écart prouvant une réactivité de la vanne EGR à des conditions de fonctionnement différentes détectées et gérées par le calculateur de gestion moteur (sans capteur NOx). Le schéma de ce protocole est illustré à la fig. 3.
A la phase 1, moteur sans charge, on compare les valeurs mesurées avec les valeurs données par l'EOBD, pour différents niveaux de d'émission de NOx (A : niveau haut ; B : niveau médium ; C : niveau faible et D : niveau extra faible ; niveau N : valeur OEBD).
On répète les mesure à la phase 2, pour un moteur en faible charge (obstacle). Si on observe des différences Delta 1, 2 et 3, alors on conclut que le système de régulation fonctionne.

**EXEMPLE 5 : Contrôle des moteurs Euro 6**

Pour les moteurs Euro 6, le principe est plus simple. En effet, ces moteurs sont dotés d'une sonde NOx dont les informations sont exploitées par le calculateur. Ces informations sont disponibles via une connexion EOBD auprès du calculateur. Le protocole de mesure cherche à identifier si les valeurs vues du calculateur sont homogènes et cohérentes avec la réalité, par une comparaison entre information interne du véhicule et mesure physique en sortie de pot d'échappement.
Le schéma de ce protocole est illustré à la fig. 4. On observe provoque l'ouverture et la fermeture de la vanne en changeant le régime (ralenti ou accéléré) et on mesure les valeurs de NOx émis. On les compare aux valeurs données par l'ordinateur de bord (EOBD). Si elles correspondent, le système fonctionne.

**Revendications**

1. Dispositif de mesure simultanée de l'opacité et de la teneur en oxydes d'azote d'un gaz d'échappement d'un véhicule automobile à moteur thermique, le dispositif comprenant une enceinte (1) à paroi tubulaire (11) de longueur *Le* déterminée, fermée à ses extrémités (14, 15), présentant un conduit d'entrée (3) et au moins un conduit de sortie (4) pour la circulation dudit gaz dans l'enceinte, comportant :

   a) des moyens d'émission (5) d'un faisceau lumineux traversant l'enceinte (1) dans sa longueur, des moyens de réception (6) dudit faisceau lumineux ayant traversé l'enceinte, et des moyens de transformation de celui-ci en un premier signal électrique proportionnel à l'intensité lumineuse reçue, pour donner une mesure de l'opacité du gaz présent dans l'enceinte,
   le dispositif étant **caractérisé en ce qu'**il comprend
   b) des moyens de détection (2) des oxydes d'azote NOx logés au moins partiellement dans une cavité (13) ménagée dans la paroi (11) de l'enceinte, hors du trajet dudit faisceau lumineux, délivrant un second signal électrique proportionnel à la quantité d'oxydes d'azote détectée, pour donner une mesure de la teneur en oxydes d'azote du gaz présent dans l'enceinte, et

c) des moyens de transmission à une unité centrale et de traitement desdits premier et second signaux électriques.

2. Dispositif de mesure selon la revendication 1, **caractérisé en ce que** les moyens d'émission (5) et de réception (6) du faisceau lumineux sont disposés à une première extrémité (14) de l'enceinte (1), et des moyens de réflexion (7) dudit faisceau sont disposés à une seconde extrémité (15) de l'enceinte (1), de sorte que le faisceau lumineux émis traverse deux fois l'enceinte, selon une trajectoire directe ($F_D$) et une trajectoire retour ($F_R$), avant d'atteindre lesdits moyens de réception.

3. Dispositif de mesure selon la revendication 1 ou 2, **caractérisé en ce que** l'enceinte (1) comprend, dans le prolongement dudit au moins un conduit de sortie (4), une prise d'air (8) munie d'un ventilateur (9) pour créer un flux gazeux traversant l'enceinte (1) perpendiculairement à son axe entre ladite prise d'air et ledit conduit de sortie.

4. Dispositif de mesure selon la revendication précédente, **caractérisé en ce que** l'enceinte (1) comprend deux ensembles, chacun formé d'une prise d'air (8) et d'un conduit de sortie (4) en prolongement, lesdits ensembles étant placés de part et d'autre du conduit d'entrée (3) et définissant une chambre (12) de circulation des gaz de longueur définie *Lc*.

5. Dispositif de mesure selon la revendication précédente, **caractérisé en ce que** la longueur *Lc* de la chambre de circulation des gaz (12) est inférieure à la longueur *Le* de l'enceinte (1), celle-ci comportant à ses première et seconde extrémités (14, 15) respectivement une première et une seconde zones terminales (24, 25) libres de gaz d'échappement.

6. Dispositif de mesure selon l'une des revendications 2 à 5, **caractérisé en ce que** les moyens d'émission (5) et de réception (6) sont disposés dans un caisson (18) ménagé à la première extrémité (14) de l'enceinte (1), sur deux parois dudit caisson orientées selon des plans perpendiculaires, et une lame séparatrice (17) est placée dans un plan orienté à 45° par rapport auxdites deux parois sur la trajectoire directe et sur la trajectoire retour du faisceau lumineux, de sorte que chacun desdits faisceaux direct ($F_D$) et retour ($F_R$) est séparé en un faisceau transmis (ft) et un faisceau dévié (fd) d'intensités relatives fixes.

7. Dispositif de mesure selon l'une quelconque des revendications précédentes, **caractérisé en ce que** les moyens de détection (2) des oxydes d'azote comprennent un capteur NOx dont l'élément sensible (2a) est à base de céramique d'un oxyde métallique, muni d'une paire d'électrodes mesurant une différence de potentiel ou un courant proportionnel à la concentration en NOx dans le gaz d'échappement.

8. Dispositif de mesure selon la revendication précédente, **caractérisé en ce que** ledit capteur NOx et ledit conduit d'entrée (3) du gaz d'échappement sont placés à la partie médiane de l'enceinte (1), la cavité logeant le capteur NOx étant ménagée dans la paroi supérieure de l'enceinte et l'élément sensible (2a) dudit capteur NOx affleurant du plan de la paroi (11) de l'enceinte, tandis que le conduit d'entrée débouche dans l'enceinte selon une orientation sensiblement horizontale.

9. Dispositif de mesure selon l'une des revendications précédentes, **caractérisé en ce que** les moyens de traitement desdits premier et second signaux électriques comprennent

   a) des moyens de stockage et d'impression de la valeur de l'opacité et de la teneur en NOx dudit gaz d'échappement, mesurées à un même instant de fonctionnement dudit véhicule automobile, et/ou
   b) des moyens pour comparer les valeurs d'opacité et de teneur en NOx mesurées avec des valeurs calculées par un système électronique régulant les émissions de NOx dudit véhicule automobile.

10. Procédé de mesure simultanée de l'opacité et de la teneur en oxydes d'azote d'un gaz d'échappement émis par un véhicule automobile à moteur thermique, **caractérisé en ce qu'il** comprend les étapes consistant à :

   - faire circuler ledit gaz d'échappement dans l'enceinte d'un dispositif de mesure simultanée de l'opacité et de la teneur en oxydes d'azote, le dispositif comprenant une enceinte (1) à paroi tubulaire (11) de longueur *Le* déterminée, fermée à ses extrémités (14, 15) et présentant un conduit d'entrée (3) et au moins un conduit de sortie (4) pour la circulation dudit gaz dans l'enceinte,
   - émettre un faisceau lumineux à travers l'enceinte (1) dans sa longueur depuis une source lumineuse (5) jusqu'à un récepteur (6), pour fournir un premier signal électrique proportionnel à l'intensité lumineuse reçue,

- réaliser la détection des oxydes d'azote par des moyens de détection (2) logés au moins partiellement dans une cavité (13) ménagée dans la paroi (11) de l'enceinte (1), hors du trajet dudit faisceau lumineux, délivrant un second signal électrique proportionnel à la quantité d'oxydes d'azote détectée,

pour obtenir une mesure de l'opacité et une mesure de la teneur en oxydes d'azote du gaz d'échappement présent dans l'enceinte à un même instant du fonctionnement dudit véhicule automobile.

11. Procédé de mesure selon la revendication 10, **caractérisé en ce que** le faisceau lumineux émis traverse deux fois l'enceinte (1), selon une trajectoire directe ($F_D$) et une trajectoire retour ($F_R$), avant d'atteindre les moyens de réception (6).

12. Procédé de mesure selon la revendication précédente, **caractérisé en ce que** le gaz d'échappement circule dans l'enceinte (1) depuis le conduit d'entrée (3) jusqu'à deux conduits de sortie (4) placés de part et d'autre dudit conduit d'entrée, de l'air étant introduit dans l'enceinte à proximité des deux conduits de sortie par deux prises d'air (8) et pulsé par deux ventilateurs (9) pour créer deux écoulements laminaires traversant l'enceinte perpendiculairement à son axe et définissant une chambre de circulation des gaz de longueur définie *Lc*, inférieure à la longueur *Le* de l'enceinte.

13. Procédé de mesure selon l'une des revendications 10 à 12, **caractérisé en ce que** le faisceau lumineux est émis par une source lumineuse (5) et est reçu par un récepteur (6) qui sont placés dans une première zone terminale (24) ménagée à une première extrémité de l'enceinte (1), et le faisceau lumineux est réfléchi par un miroir plan qui est placé dans une seconde zone terminale (25) ménagée à une seconde extrémité de l'enceinte, lesdites première et seconde zones terminales étant libres de gaz d'échappement.

14. Procédé de mesure selon l'une des revendications 10 à 13, **caractérisé en ce que** les oxydes d'azote sont détectés par un capteur NOx affleurant du plan de la paroi (11) de l'enceinte (1) à proximité du conduit d'entrée (3) des gaz d'échappement, lequel débouche dans l'enceinte dans sa partie médiane, avec un temps de réponse inférieur à une seconde.

15. Procédé de mesure selon l'une des revendications 10 à 14, **caractérisé en ce que** lesdits premier et second signaux électriques sont transmis à une unité centrale et sont traités (a) par stockage et impression de la valeur de l'opacité et de la teneur en NOx dudit gaz d'échappement, mesurées à un même instant de fonctionnement dudit véhicule automobile, et/ou (b) par comparaison des valeurs d'opacité et de teneur en NOx mesurées, avec des valeurs calculées par un système électronique régulant les émissions de NOx dudit véhicule automobile.

**Patentansprüche**

1. Vorrichtung zum gleichzeitigen Messen der Opazität und des Stickoxidgehalts eines Abgases eines Kraftfahrzeugs mit Verbrennungsmotor, wobei die Vorrichtung einen abgegrenzten Raum (1) mit röhrenförmiger Wand (11) umfasst, der eine festgelegte Länge Le hat und an seinen Enden (14, 15) geschlossen ist, wobei er eine Eintrittsleitung (3) und mindestens eine Austrittsleitung (4) umfasst, damit das Gas durch den abgegrenzten Raum strömen kann, und wobei sie Folgendes aufweist:

a) Mittel zum Aussenden (5) eines Lichtstrahls, der den abgegrenzten Raum (1) seiner Länge nach durchquert, Mittel zum Empfangen (6) des Lichtstrahls, der den abgegrenzten Raum durchquert hat, und Mittel zum Umwandeln desselben in ein erstes elektrisches Signal, das proportional zur empfangenen Lichtintensität ist, um einen Messwert der Opazität des Gases zu erhalten, welches in dem abgegrenzten Raum vorliegt, wobei die Vorrichtung **dadurch gekennzeichnet ist, dass** sie Folgendes umfasst:

b) Mittel zum Detektieren (2) von Stickoxiden $NO_x$, welche mindestens teilweise in einem Hohlraum (13) angeordnet sind, der außerhalb der Durchlaufstrecke des Lichtstrahls in der Wand (11) des abgegrenzten Raums ausgespart ist, wobei diese ein zweites elektrisches Signal liefern, das proportional zur detektierten Menge an Stickoxiden ist, um einen Messwert des Gehaltes an Stickoxiden des Gases zu erhalten, welches in dem abgegrenzten Raum vorliegt, und

c) Mittel zum Übertragen an einen Hauptprozessor, der auch zum Verarbeiten des ersten und des zweiten elektrischen Signals dient.

2. Messvorrichtung nach Anspruch 1, **dadurch gekennzeichnet, dass** die Mittel zum Aussenden (5) und zum Empfangen (6) des Lichtstrahls an einem ersten Ende (14) des abgegrenzten Raums (1) angeordnet sind, und Mittel zum Reflektieren (7) des Strahls an einem zweiten Ende (15) des abgegrenzten Raums (1) angeordnet sind, sodass der ausgesendete Lichtstrahl den abgegrenzten Raum zweimal durchläuft, gemäß einer direkten Laufstrecke ($F_D$) und einer Rücklaufstrecke ($F_R$), bevor er die Empfangsmittel erreicht.

3. Messvorrichtung nach Anspruch 1 oder 2, **dadurch gekennzeichnet, dass** der abgegrenzte Raum (1) einen Lufteinlass (8) umfasst, der in der Verlängerung der mindestens einen Austrittsleitung (4) gelegen ist und der mit einem Ventilator (9) versehen ist, um einen Gasstrom zu erzeugen, welcher den abgegrenzten Raum (1) rechtwinklig zu dessen Achse vom Lufteinlass zur Austrittsleitung durchströmt.

4. Messvorrichtung nach dem vorhergehenden Anspruch, **dadurch gekennzeichnet, dass** der abgegrenzte Raum (1) zwei Gesamteinheiten umfasst, wobei jede davon von einem Lufteinlass (8) und einer sich davon anschließenden Austrittsleitung (4) gebildet wird, wobei die Gesamteinheiten beiderseits der Eintrittsleitung (3) angeordnet sind und eine Kammer (12) mit festgelegter Länge *Lc* örtlich bestimmen, in welcher die Gase strömen.

5. Messvorrichtung nach dem vorhergehenden Anspruch, **dadurch gekennzeichnet, dass** die Länge *Lc* der Gasströmungskammer (12) geringer als die Länge Le des abgegrenzten Raums (1) ist, da diese an ihrem ersten und zweiten Ende (14, 15) einen ersten beziehungsweise einen zweiten Endbereich (24, 25) aufweist, welche frei von Abgas sind.

6. Messvorrichtung nach einem der Ansprüche 2 bis 5, **dadurch gekennzeichnet, dass** die Mittel zum Aussenden (5) und Empfangen (6) in einem Kasten (18) angeordnet sind, der am ersten Ende (14) des abgegrenzten Raums (1) eingerichtet ist, und zwar an zwei Wänden des Kastens, welche gemäß rechtwinklig zueinander stehenden Ebenen ausgerichtet sind, und eine Trennscheibe (17) in einer Ebene angeordnet ist, die gemäß einem 45°-Winkel zu den beiden Wänden ausgerichtet ist und in der direkten Laufstrecke und in der Rücklaufstrecke des Lichtstrahl liegt, sodass sowohl der direkte ($F_D$) als auch der Rücklaufstrahl ($F_R$) in einen durchgehenden Strahl (ft) und einen abgelenkten Strahl (fd) aufgetrennt werden, wobei deren Intensitäten verhältnismäßig geringen Schwankungen unterliegen.

7. Messvorrichtung nach einem beliebigen der vorhergehenden Ansprüche, **dadurch gekennzeichnet, dass** die Mittel zum Detektieren (2) von Stickoxiden einen NOx-Sensor umfassen, dessen Messfühlerelement (2a) auf einer Keramik aus einem Metalloxid beruht, wobei es mit einem Elektrodenpaar versehen ist, welches eine Potenzialdifferenz oder einen Strom misst, die/der proportional zur Konzentration an NOx im Abgas ist.

8. Messvorrichtung nach dem vorhergehenden Anspruch, **dadurch gekennzeichnet, dass** der NOx-Sensor und die Abgaseintrittsleitung (3) im mittleren Bereich des abgegrenzten Raums (1) angeordnet sind, wobei der Hohlraum, in welchem der NOx-Sensor aufgenommen ist, in der oberen Wand des abgegrenzten Raums eingerichtet ist und das Messfühlerelement (2a) des NOx-Sensors bündig mit der Ebene der Wand (11) des abgegrenzten Raums abschließt, während die Eintrittsleitung gemäß einer im Wesentlichen horizontalen Ausrichtung in den abgegrenzten Raum einmündet.

9. Messvorrichtung nach einem der vorhergehenden Ansprüche, **dadurch gekennzeichnet, dass** die Mittel zur Verarbeitung des ersten und des zweiten elektrischen Signals Folgendes umfassen:

a) Mittel zum Speichern und Drucken der Werte der Opazität und des $NO_x$-Gehalts des Abgases, wobei diese zu ein und demselben Betriebszeitpunkt des Kraftfahrzeugs gemessen werden, und/oder
b) Mittel zum Vergleichen der gemessenen Werte der Opazität und des NOx-Gehalts mit Werten, die von einem elektronischen System berechnet wurden, welches den NOx-Ausstoß des Kraftfahrzeugs reguliert.

10. Verfahren zum gleichzeitigen Messen der Opazität und des Stickoxidgehalts eines Abgases, das von einem Kraftfahrzeug mit Verbrennungsmotor ausgestoßen wird, **dadurch gekennzeichnet, dass** es die folgenden Schritte umfasst:

- Einleiten der Abgase in den abgegrenzten Raum einer Vorrichtung zum gleichzeitigen Messen der Opazität und des Stickoxidgehalts, wobei die Vorrichtung einen abgegrenzten Raum (1) mit röhrenförmiger Wand (11) umfasst, der eine festgelegte Länge Le hat und an seinen Enden (14, 15) geschlossen ist, wobei er eine Eintrittsleitung (3) und mindestens eine Austrittsleitung (4) umfasst, damit das Gas durch den abgegrenzten Raum strömen kann,
- Aussenden eines Lichtstrahls, der den abgegrenzten Raum (1) ausgehend von einer Lichtquelle (5) bis zu einem Empfänger (6) durchläuft, um ein erstes elektrisches Signal bereitzustellen, das proportional zur empfangenen Lichtintensität ist,

- Durchführen der Detektion der Stickoxide mit Hilfe von Detektionsmitteln (2), die mindestens teilweise in einem Hohlraum (13) angeordnet sind, der außerhalb der Durchlaufstrecke des Lichtstrahls in der Wand (11) des abgegrenzten Raums (1) eingerichtet ist, wobei sie ein zweites elektrisches Signal liefern, das proportional zur detektierten Menge an Stickoxiden ist,

um einen Messwert der Opazität und einen Messwert des Stickoxidgehalts für das Abgas zu erhalten, welches zu ein- und demselben Betriebszeitpunkt des Kraftfahrzeugs in dem abgegrenzten Raum vorliegt.

11. Messverfahren nach Anspruch 10, **dadurch gekennzeichnet, dass** der ausgesendete Lichtstrahl den abgegrenzten Raum (1) zweimal durchläuft, gemäß einer direkten Laufstrecke ($F_D$) und einer Rücklaufstracke ($F_R$), bevor er die Empfangsmittel (6) erreicht.

12. Messverfahren nach dem vorhergehenden Anspruch, **dadurch gekennzeichnet, dass** das Abgas innerhalb des abgegrenzten Raumes (1) von der Eintrittsleitung (3) zu zwei Austrittsleitungen (4) strömt, die beiderseits der Eintrittsleitung angeordnet sind, wobei in der Nähe der beiden Austrittsleitungen durch zwei Lufteinlässe (8) Luft in den abgegrenzten Raum eingeleitet und von zwei Ventilatoren (9) in Bewegung gesetzt wird, um zwei Laminarströmungen zu erzeugen, die den abgegrenzten Raum rechtwinklig zu dessen Achse durchqueren und eine Gasströmungskammmer örtlich bestimmen, deren Länge Lc festgelegt ist, wobei sie geringer als die Länge Le des abgegrenzten Raums ist.

13. Messverfahren nach einem der Ansprüche 10 bis 12, **dadurch gekennzeichnet, dass** der Lichtstrahl von einer Lichtquelle (5) ausgesendet wird und von einem Empfänger (6) empfangen wird, wobei diese in einem ersten Endbereich (24) angeordnet sind, der an einem ersten Ende des abgegrenzten Raums (1) eingerichtet ist, und der Lichtstrahl von einem ebenen Spiegel reflektiert wird, wobei dieser in einem zweiten Endbereich (25) angeordnet ist, der an einem zweiten Ende des abgeschlossenen Raums eingerichtet ist, wobei der erste und der zweite Endbereich frei von Abgas sind.

14. Messverfahren nach einem der Ansprüche 10 bis 13, **dadurch gekennzeichnet, dass** die Stickoxide von einem NOx-Sensor detektiert werden, der bündig mit der Ebene der Wand (11) des abgegrenzten Raums (1) abschließt, in der Nähe der Abgaseintrittsleitung (3), welche im mittleren Abschnitt des abgegrenzten Raums in diesen einmündet, mit einer Ansprechzeit von weniger als einer Sekunde.

15. Messverfahren nach einem der Ansprüche 10 bis 14, **dadurch gekennzeichnet, dass** das erste und das zweite elektrische Signal an einen Hauptprozessor übertragen und verarbeitet werden, indem a) der Wert der Opazität und des NOx-Gehalts des Abgases, gemessen zu ein- und demselben Betriebszeitpunkt des Kraftfahrzeugs, gespeichert und ausgedruckt werden und/oder b) indem die gemessenen Werte der Opazität und des NOx-Gehalts mit Werten verglichen werden, die von einem elektronischen System berechnet wurden, welches den NOx-Ausstoß des Kraftfahrzeugs reguliert.

**Claims**

1. Device for simultaneously measuring the opacity and the content of nitrogen oxides of an exhaust gas of a motor vehicle with a combustion engine, the device comprising an enclosure (1) having a tubular wall (11) of determined length Le, sealed at its ends (14, 15), having an inlet duct (3) and at least one outlet duct (4) for the circulation of said gas in the enclosure, comprising:

a) means (5) for emitting a light beam that passes through the enclosure (1) along its length, means (6) for receiving said light beam that has passed through the enclosure, and means for converting this light beam into a first electrical signal proportional to the light intensity received, in order to give a measurement of the opacity of the gas present in the enclosure,
the device being **characterized in that** it comprises:
b) means (2) for detecting nitrogen oxides NOx lodged at least partially in a cavity (13) made in the wall (11) of the enclosure, out of the path of said light beam, that deliver a second electrical signal proportional to the amount of nitrogen oxides detected, in order to give a measurement of the content of nitrogen oxides of the gas present in the enclosure, and
c) means for transmitting said first and second electrical signals to a central processing unit.

2. Measuring device according to Claim 1, **characterized in that** the light beam emitting means (5) and receiving means (6) are positioned at a first end (14) of the enclosure (1), and means (7) for reflecting said beam are positioned at a second end (15) of the enclosure (1), so that the light beam emitted passes through the enclosure twice, along a direct trajectory ($F_D$) and a return trajectory ($F_R$), before reaching said receiving means.

3. Measuring device according to Claim 1 or 2, **characterized in that** the enclosure (1) comprises, in the extension of said at least one outlet duct (4), an air intake (8) equipped with a fan (9) in order to create a gas flow that passes through the enclosure (1) perpendicular to its axis between said air intake and said outlet duct.

4. Measuring device according to the preceding claim, **characterized in that** the enclosure (1) comprises two assemblies, each formed of an air intake (8) and of an outlet duct (4) extending therefrom, said assemblies being placed on either side of the inlet duct (3) and defining a gas circulation chamber (12) of defined length Lc.

5. Measuring device according to the preceding claim, **characterized in that** the length *Lc* of the gas circulation chamber (12) is smaller than the length *Le* of the enclosure (1), the latter comprising at its first and second ends (14, 15) respectively first and second terminal zones (24, 25) free of exhaust gas.

6. Measuring device according to one of Claims 2 to 5, **characterized in that** the emitting means (5) and receiving means (6) are positioned in a housing (18) arranged at the first end (14) of the enclosure (1), on two walls of said housing oriented along perpendicular planes, and a beam splitter (17) is placed in a plane oriented at 45° with respect to said two walls through the direct trajectory and through the return trajectory of the light beam, so that each of said direct ($F_D$) and return ($F_R$) beams is separated into a transmitted beam (ft) and a deflected beam (fd) of fixed relative intensities.

7. Measuring device according to any one of the preceding claims, **characterized in that** the means (2) for detecting nitrogen oxides comprise an NOx sensor, the sensing element (2a) of which is based on a metal oxide ceramic, equipped with a pair of electrodes that measure a potential difference or a current proportional to the concentration of NOx in the exhaust gas.

8. Measuring device according to the preceding claim, **characterized in that** said NOx sensor and said inlet duct (3) of the exhaust gas are placed in the median portion of the enclosure (1), the cavity housing the NOx sensor being arranged in the upper wall of the enclosure and the sensing element (2a) of said NOx sensor being flush with the plane of the wall (11) of the enclosure, whilst the inlet duct opens into the enclosure along a substantially horizontal orientation.

9. Measuring device according to one of the preceding claims, **characterized in that** the means for processing said first and second electrical signals comprise:

a) means for storing and printing the value of the opacity and of the NOx content of said exhaust gas, measured at one and the same moment of operation of said motor vehicle, and/or
b) means for comparing the measured opacity and NOx content values with values calculated by an electronic system that regulates the NOx emissions of said motor vehicle.

10. Process for simultaneously measuring the opacity and the content of nitrogen oxides of an exhaust gas emitted by a motor vehicle with a combustion engine, **characterized in that** it comprises the steps consisting in:

- circulating said exhaust gas in the enclosure of a device for simultaneously measuring the opacity and the content of nitrogen oxides, the device comprising an enclosure (1) having a tubular wall (11) of determined length *Le*, sealed at its ends (14, 15) and having an inlet duct (3) and at least one outlet duct (4) for the circulation of said gas in the enclosure,
- emitting a light beam through the enclosure (1) along its length from a light source (5) to a receiver (6), in order to provide a first electrical signal proportional to the light intensity received,
- detecting the nitrogen oxides using detection means (2) lodged at least partially in a cavity (13) made in the wall (11) of the enclosure (1), out of the path of said light beam, that deliver a second electrical signal proportional to the amount of nitrogen oxides detected,
in order to obtain a measurement of the opacity and a measurement of the content of nitrogen oxides of the exhaust gas present in the enclosure at one and the same moment of operation of said motor vehicle.

**11.** Measuring process according to Claim 10, **characterized in that** the light beam emitted passes through the enclosure (1) twice, along a direct trajectory ($F_D$) and a return trajectory ($F_R$), before reaching the receiving means (6).

**12.** Measuring process according to the preceding claim, **characterized in that** the exhaust gas circulates in the enclosure (1) from the inlet duct (3) to two outlet ducts (4) placed on either side of said inlet duct, air being introduced into the enclosure in the vicinity of the two outlet ducts by two air intakes (8) and driven by two fans (9) in order to create two laminar flows that pass through the enclosure perpendicular to its axis and that define a gas circulation chamber of defined length *Lc*, shorter than the length Le of the enclosure.

**13.** Measuring process according to one of Claims 10 to 12, **characterized in that** the light beam is emitted by a light source (5) and is received by a receiver (6) which are placed in a first terminal zone (24) arranged at a first end of the enclosure (1), and the light beam is reflected by a flat mirror which is placed in a second terminal zone (25) arranged at a second end of the enclosure, said first and second terminal zones being free of exhaust gas.

**14.** Measuring process according to one of Claims 10 to 13, **characterized in that** the nitrogen oxides are detected by a NOx sensor that is flush with the plane of the wall (11) of the enclosure (1) in the vicinity of the inlet duct (3) of the exhaust gases, which opens into the enclosure in its median portion, with a response time of less than one second.

**15.** Measuring process according to one of Claims 10 to 14, **characterized in that** said first and second electrical signals are transmitted to a central unit and are processed (a) by storing and printing the value of the opacity and of the NOx content of said exhaust gas, measured at one and the same moment of operation of said motor vehicle, and/or (b) by comparing the measured opacity and NOx content values with values calculated by an electronic system that regulates the NOx emissions of said motor vehicle.

Fig.1

Fig.2a

Fig.2 b

Phase 1

Phase 2

Figure 3

: Vanne EGR fermée

: Vanne EGR ouverte

1 : Delta 1
2 : Delta 2
3 : Delta 3

Figure 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2703460 A1

- WO 2011143304 A2